# EUROPEAN PATENT APPLICATION

(11) **EP 4 443 218 A1**
(43) Date of publication of application: **09.10.2024**
(21) Application number: 24168445.5
(22) Date of filing: 04.04.2024
(51) Int. Cl.: G02C 7/02, G02C 7/06, G02C 13/00

(54) **CALCULATION MODULE, SYSTEM AND METHOD FOR DETERMINING PRODUCTION PARAMETERS OF AN OPTICAL ELEMENT**

(30) Priority: 05.04.2023 EP 23305494
(71) Applicant: Essilor International, 94220 Charenton-Le-Pont (FR)
(72) Inventor: ENCAOUA, David, 94220 CHARENTON-LE-PONT (FR); PICARD, Stéphane, 94220 CHARENTON-LE-PONT (FR)
(74) Representative: Korakis-Ménager, Sophie

(57) **Abstract**

A calculation module for determining a probability model of a parameter of a first reference posture of a subject, the probability model being intended to determine at least a production parameter of an optical element suitable for the subject, the calculation module being configured: to determine at least one value of a parameter of a second reference posture, during an occurrence of the second reference posture, and to determine the probability model of a parameter of a first reference posture based on the at least one value of the parameter of the second reference posture.

## Description

### Technical field

This disclosure relates to calculation module, system, and method for determining a probability model of a parameter of a reference posture of a subject. The probability model is intended to determine at least a production parameter of an optical element suitable for the subject, in particular optical element having multiple areas to be parametrized.

### Background information and prior art

**The** disclosure relates to the analysis of the posture of the subject during a specific visual task, for the purposes of personalisation and/or optimisation of the optical characteristics of an optical element intended to be in front of the subject or to be worn by the subject, as well as their mounting on the frame. The optical element may be for example corrective lenses or ophthalmic lenses which the subject could wear. The optical element may be a screen in front of the subject. By posture, one means the position of the head of the subject and/or the eyes of the subject, or other parts of the subject during a specific task. One also means by posture, the position of virtual axes representing for example the glare axis of the subject with respect to the optical element.

More precisely, the disclosure may relate to the analysis of the posture of the head and the eyes of the subject doing specific tasks and related to measurements to obtain data necessary for determining the manufacturing parameter of an optical element with respect to the eyes of the subject and for personalizing and optimising the optical characteristics of optical element. The posture of the head and the eyes of the subject doing specific tasks in respect to a specific protocol is called a reference posture, the reference posture being used to obtain specific data necessary for determining the manufacturing parameter of the optical element.

Numerous systems, aiming at optimising the position of lenses in a frame relative to the relative position of the pupils of the eyes of the subject and of the frame, are known already. For this purpose, fixed or animated images of the face wearing the frame are taken by a camera, and detection of the position of the eyes is carried out, as is detection of the placement of the frame.

For example, PCT application WO2011161087A1, filed by the applicant, discloses a system in which, from a series of animated images of the face of the subject during movement in front of a fixed camera, a reference posture is determined when the subject gazes a target at a given position. In particular, the reference posture is determined by making multiple measurements of the reference posture with one given target position, in order to statistically estimate the optimal subject's reference posture when the subject gazes a target at a given position. During the process, the subject will alternate between gaze the target at the given position and a gaze in a different direction and/or distance, away from the given target position. The aim is to analyse how the wearer gets back to the reference posture after looking at something else (on the sides, up or down). With this method, only one reference posture is determined. To determine a second reference posture when the subject gazes the target at another position, another measurement with a different protocol must be done another time. The succession of protocols and measurements at different position is long and tedious for the subject who can get tired of the repetition, which can modify his head posture.

Thus, there is a need to a more reliable and accurate measurement and to reduce the time for determining parameters of several reference postures of a subject, the reference posture, allowing to determine at least a production parameter of an optical element suitable for the subject, in particular optical element having multiple areas to be parametrized.

### Summary

The following presents a simplified summary to provide a basic understanding of various aspects of this disclosure. This summary is not an extensive overview of all contemplated aspects and is intended to neither identify key or critical elements of all aspects nor delineate the scope of any or all aspects. The sole purpose is to present some concepts of one or more aspects in a simplified form as a prelude to the more detailed description that is presented later.

One aspect of this disclosure is a calculation module for determining a probability model of a parameter of a first reference posture of a subject. The probability model is intended to determine at least a production parameter of an optical element suitable for the subject. The calculation module is configured to determine at least one value of a parameter of a second reference posture, during an occurrence of the second reference posture and to determine the probability model of a parameter of a first reference posture based on the at least one value of the parameter of the second reference posture and based on a model of constraints.

The model of constraints is a parametric model having as parameter at least parameters of the second reference posture.

The inventors shown that it is possible to obtain a probability model of a parameter of a first reference posture from a model of constraints and at least a value of parameter of a second reference posture. Consequently, the present disclosure has the advantage to obtain a more reliable and accurate measurement for determining parameters of the reference postures of the subject.

When making an optical element such as a progressive lens, it is needed to precisely position the optical centre of the lens, which is usually a position for far vision, and needed to determine on the optical element an optimal point for near vision. These two points, as well as others (intermediate vision for example) depend on the visual behaviour, preferences, and habits of the wearer. The reliable and accurate measurement of these points is a challenge for the optician, and when only far vision position is measured, it's challenging for the lens manufacturer to calculate the best fit for near vision position. Thanks to the present disclosure, the two points are obtained faster, in a reliable and accurate way, from a measurement of the parameter of the reference posture and the probability model determined based on the at least one value of the parameter of the second reference posture and based on a model of constraints.

According to further embodiments, which can be considered alone or in combination, the calculation module is configured further to determine at least one value of the parameter of the first reference posture during an occurrence of the first reference posture, and to determine the probability model based on at least one value of the parameter of the first reference posture and the at least one value of the parameter of the second reference posture.

Advantageously, the determining of the first and the second reference allows the switching between the two references to be analysed more precisely.

According to further embodiments, which can be considered alone or in combination, the calculation module may configure further to determine at least one value of the parameter of the supplementary reference posture during an occurrence of the supplementary posture, and to determine the probability model based on at least one value of the parameter of the first reference posture , the at least one value of the parameter of the second reference posture and on at least one value of the parameter of the supplementary posture. There may be one, two or more supplementary posture.

**The** supplementary reference posture may be a third reference posture or more. The supplementary reference posture may be different than first and second reference posture.

According to further embodiments, which can be considered alone or in combination, the step of determining a first and/or a second reference posture may be obtained by using the method described in WO2011161087A1. Advantageously, the determining is more reliable and accurate.

According to further embodiments, which can be considered alone or in combination, the step of determining the probability model is based on the comparison between the at least one value of the parameter of the first reference posture and the at least one value of the parameter of the second reference posture.

According to further embodiments, which can be considered alone or in combination, the model of constraints may be a model of design constraints and/or a model of behaviour constraints.

The model of design constraints may be a model based on the design of the optical element intended to be manufactured. In this embodiment, the optical element is a progressive optical element. The parameters of the model of design constraints may be parameters of the progression curve of corridor of the optical element (progression curve means the spatial function of the addition in the progression corridor). For example the parameters of the progression curve of corridor of the optical element may be a length of corridor of the optical element. The value of the length of corridor may be a standard value (for example 18mm if the parameter is the length of corridor) or an input value (between 10 mm and 20mm or between 14mm or 18mm). The input value may be provided by the ECP, by the lens manufacturer or by the subject. The input value may be obtained by other measurements.

The model of behaviour constraint may be a theorical model or experimental model based on collected data.

The behaviour model may be a theorical model or experimental model based on collected data.

According to further embodiments, which can be considered alone or in combination, the first and/or second reference posture (FV) being characterized by an orientation of a head of the subject relatively to a gaze axis of the subject.

According to further embodiments, which can be considered alone or in combination, the parameter of the first and/or the second reference posture being a cap angle value and/or a frontal angle value (FV: panto). By cap angle, one mean angle relatively to a longitudinal axis relatively to the head, the longitudinal the axis being close to the vertical when the subject is standing in primary position. By frontal angle in far vision, we mean pantoscopic angle as described in ISO13666.

According to further embodiments, which can be considered alone or in combination, the parameter of the first and/or second reference posture represents a location of the optical element crossed by a gaze axis of the subject while respectively the first and/or second reference posture.

According to further embodiments, which can be considered alone or in combination, the step of determining the probability model is based at least on the difference between the first reference posture and the second reference posture.

The difference may be the difference between the value of the parameter of the first reference posture and the second reference posture such as the orientation of the head of the subject, the cap angle, the frontal angle or the location of the optical element crossed by a gaze axis of the subject while the first and second reference posture. According to further embodiments, which can be considered alone or in combination, the calculation module comprises further, based on the probability model, determine a zone of at least one parameter of the first reference posture, the values of the at least one parameter of the first reference posture being superior to a given probability threshold.

According to further embodiments, which can be considered alone or in combination, the calculation module comprises further based on the probability model, determine a representing point. The representing point may be a maximal probability in the zone or a pondered average of the probability in the zone.

The representing point (FV) being located in the vicinity of the first reference posture (FV) when the difference is comprised between a first difference threshold and a second difference threshold.

The representing (FV) being located above the first reference posture (FV) when the difference is lower than the first difference threshold.

**The** representing point (FV) being located below the first reference posture (FV) when the difference is higher than the second difference threshold.

According to further embodiments, which can be considered alone or in combination, the calculation module is also configured to obtain a location of an adapted point (FV), the adapted point (FV) being one of the points of the probability model (FV), the location of the adapted point (FV) being intended to determine the value of the manufacturing parameter of the optical element.

According to further embodiments, which can be considered alone or in combination, the calculation module is configured to obtain the location of the adapted point (FV) by selecting an adapted point (FV) among points of the zone (FV), for example by selecting the representing point (FV) or the calculation being configured to command a display device to display the zone (FV) and to obtain from an input device the location of the adapted point (FV).

According to further embodiments, which can be considered alone or in combination, the first reference posture (FV) corresponding to a far vision posture of the subject and/or the second reference posture (NV) corresponds to a near vision posture of the subject.

According to further embodiments, which can be considered alone or in combination, the first reference posture corresponds to a posture in which the subject naturally gazes at a first target and/or the second reference posture corresponds to a posture in which the subject naturally gazes at a second target.

According to further embodiments, which can be considered alone or in combination, the frontal angle value and the cap angle value characterizing the first reference posture are determined by means of conditional probabilities depending on a previous first reference posture and/or a previous deviated posture of the previous first reference posture,and/or the frontal angle value and the cap angle value characterizing the second reference posture are determined by means of conditional probabilities depending on a previous second reference posture and/or a previous deviated posture of the previous second reference posture.

Advantageously, this embodiment allows reducing the time for determining parameters of the reference postures of the subject.

According to further embodiments, which can be considered alone or in combination, the calculation module is configured to obtain a first image of the subject during the occurrence of the first reference posture and to measure a location of singular points on the first image, and/or to obtain a second image of the subject during the occurrence of second first reference posture and to measure a location of singular points on the second image.

According to further embodiments, which can be considered alone or in combination, the singular points of the first image and/or the singular points of the second image being supported by an accessory fixed on an eyeglasses frame worn by the subject and/or an accessory held by the subject. Another aspect of this disclosure is a system for determining a probability model of a parameter of a first reference posture (FV) of the subject, the probability model being intended to determine at least a production parameter of an optical element suitable for the subject, the system comprising:
- a capture device to measure a second reference posture,
- a calculation module configured
   ∘ to determine at least one value of a parameter of the measured second reference posture (NV), and
   ∘ to determine the probability model of a parameter of a first reference posture (FV) based on the at least one value of the parameter of the second reference posture (NV).

Another aspect of this disclosure is a method for determining a probability model of a parameter of a first reference posture of the subject. The probability model is intended to determine at least a production parameter of an optical element suitable for the subject. The method comprises, determining at least one value of a parameter of a second reference posture and measuring the probability model of a parameter of a first reference posture based on the at least one value of the parameter of the second reference posture.

All the embodiments described for the calculation module may be applied alone or in combination to the method. In the case of the method, the steps of determining may be replaced by a of measuring a reference posture.

Another aspect of this disclosure is a computer implemented method for determining a probability model of a parameter of a first reference posture of the subject. The probability model is intended to determine at least a production parameter of an optical element suitable for the subject. The computer-implemented method comprises, determining at least one value of a parameter of a second reference posture and determining the probability model of a parameter of a first reference posture based on the at least one value of the parameter of the second reference posture.

All the embodiments described for the calculation module may be applied alone or in combination to the computer implemented method.

Another aspect of this disclosure is a computer program comprising instructions which, when the computer program is executed by a computer, cause the computer to carry out a method for determining a probability model of a parameter of a first reference posture of the subject. The probability model is intended to determine at least a production parameter of an optical element suitable for the subject. The method comprises determining at least one value of a parameter of a second reference posture and determining the probability model of a parameter of a first reference posture based on the at least one value of the parameter of the second reference posture.

All the embodiments described for the calculation module may be applied alone or in combination to the computer program.

**A** computer may include a memory and a processor. Examples of processors include microprocessors, microcontrollers, graphics processing units (GPUs), central processing units (CPUs), application processors, digital signal processors (DSPs), reduced instruction set computing (RISC) processors, systems on a chip (SoC), field-programmable gate arrays (FPGAs), programmable logic devices (PLDs), state machines, gated logic, discrete hardware circuits, and other suitable hardware configured to perform the various functionality described throughout this disclosure. The memory may be a computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor of the computer.

Another aspect of this disclosure is a non-transitory program storage device, readable by a computer, tangibly embodying a program of instructions executable by the computer to perform a method for determining a probability model of a parameter of a first reference posture of the subject. The probability model is intended to determine at least a production parameter of an optical element suitable for the subject. The method comprises determining at least one value of a parameter of a second reference posture and determining the probability model of a parameter of a first reference posture based on the at least one value of the parameter of the second reference posture.

All the embodiments described for the calculation module may be applied alone or in combination to the non-transitory program storage device.

### Description of the drawings

For a more complete understanding of the description provided herein and the advantages thereof, reference is now made to the brief descriptions below, taken in connection with the accompanying drawings and detailed description, wherein like reference numerals represent like parts.
Figure 1 represents a system for determining a probability model of a parameter of a first reference posture of a subject.
Figure 2 represents an embodiment of the system for determining a probability model of a parameter of a first reference posture of a subject.
Figures 3a and 3b represents the method, for determining a probability model of a parameter of a first reference posture of a subject.
The figures 4-a to 4-c represent examples of the determination of the first zone and determination of the location of the representing point of the first zone.
Figure 5 represents another embodiment of the method, for determining a probability model of a parameter of a first reference posture of a subject.
Figures 6-a and 6-b represent an example of the accessory held by the subject.
Figure 7 represents another embodiment of the method, for determining a probability model of a parameter of a first reference posture of a subject.
Figure 8 represents a first phase of a use case of this disclosure.
Figure 9 represents a sixth phase of the use case of this disclosure.
Figure 10 represents a tenth phase of the use case of this disclosure.

### Detailed description of embodiments

**The** detailed description set forth below in connection with the appended drawings is intended as a description of various possible embodiments and is not intended to represent the only embodiments in which the concepts described herein may be practiced. The detailed description includes specific details for the purpose of providing a thorough understanding of various concepts. However, it will be apparent to those skilled in the art that these concepts may be practiced without these specific details. In some instances, well-known structures and components are shown in block diagram form to avoid obscuring such concepts.

### Description of the system for determining a probability model of a parameter of a first reference posture of a subject

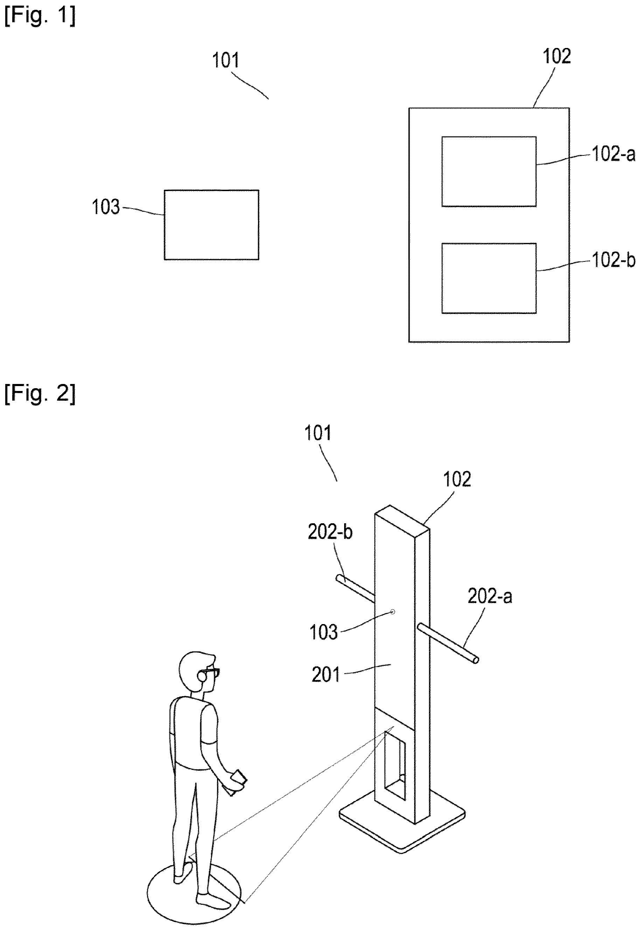

Figure 1 represents, a system 101 for determining a probability model of a parameter of a first reference posture of a subject.

The probability model of a parameter of a first reference posture represents the probability of the optimal subject's reference posture.

**The** probability model of the parameter is a mathematical representation of the values of the parameter. More precisely, the probability model associates with each possible value of the parameter a probability of this value to be optimal for the subject's reference posture. The probability model may have multiple parameters. In that case the probability model represents the probability of the reference posture to be optimal for each combination of the parameters.

The probability model may be intended to determine production parameters of the optical element.

By "production", one mean manufacturing an optical element (such as designing optically an optical element, optimizing the design of an optical element) or edging an optical element, fitting an optical element into a frame, centring or customizing an optical element.

The probability model may also be intended to determine a value of a parameter of a first zone (FV) of the optical element. The system 101 may also be configured to determine the first zone (FV) and values of parameters of the first zone (FV).

The optical element may be adapted to a subject.

**The** optical element may be an ophthalmic lens part of eyeglasses. The ophthalmic lens may comprise a correction adapted to the refraction of the subject.

The first zone (FV) may be a zone of the optical element adapted to the first reference posture (FV).

The first zone (FV) may be a zone of the optical element on which a probability of a crossing of the gaze axis during the first reference posture (FV) is superior to a first probability threshold. For example, the first probability threshold may be 60% or 50% or 40% or 30%.

The system 101 may also be configured to determine a location of an adapted point (FV), the adapted point (FV) being one of the points of the first zone (FV).

**The** first reference posture (FV) may be a far vision posture of the subject. More precisely during the far vision posture, the subject may be in a natural position and may fixe a point in infinity, right in front of him in a horizontal plane.

In this case the probability model, the value of the parameter of the first zone (FV) and/or the location of the first adapted point (FV) may be used to determine the far vision part of the optical element related to the far vision and possibly the near vision part of the optical element that are optimal to the subject.

In this disclosure one added between bracket the two letters FV (meaning Far Vision) to elements related to the first zone, the first adapted point and the first reference posture to help the reader understand this disclosure.

However, the first zone, the first adapted point and the first reference posture may be related to other visual postures than the far vision posture. The only purpose of these indications is to help the reader and not to restrict the scope of the protection as defined in the claims.

**The** value or the parameter of the zone (FV) and/or the location of the first adapted point (FV) may be intended to determine manufacturing parameters of the optical element adapted to the subject.

When the optical element is a lens of eyeglasses comprising, the correction adapted to the refraction of the subject, the probability model, the value of the parameter of the first zone (FV) and/or the location of the first adapted point (FV) can be used also for lenses centration. The lenses can be progressive lenses, and, in this case, the parameter of the first zone (FV) and/or the location of the first adapted point (FV) may be intended to optimize the length of corridor of these progressive lenses.

**The** system 101 may also be configured for determining a value of a parameter of a second zone (NV) and a location of a second adapted point (NV) of the optical element. The value of the parameter of the second zone (NV) may be adapted to a second reference posture (NV) of the subject.

The second zone (NV) may be a zone of the optical element on which a probability of a crossing of the gaze axis during the second reference posture (NV) is superior to a second probability threshold. For example, the second probability threshold may be 60% or 50% or 40% or 30%.

The second adapted point (NV) may be a point of the second zone (NV) across which a gaze axis of a subject passes through during the second reference posture (NV) of the subject.

The value of the parameter of the second zone (NV) and/or the location of the second adapted point (NV) may also be intended to determine the value of the manufacturing parameters of the optical element adapted to the subject.

The second reference posture (NV) may be a near vision posture of the subject. A near vision posture of the subject is a posture when the subject does a task in near vision. For example, the near-vision posture may be a reading position, that is, the position when the subject is reading something on a book or on a smartphone, type a message on the smartphone, or doing some specific visual tasks on a tablet. For example, if the visual task is reading a book, one subject may have a position in which the subject fixes a point at around forty centimetres from his eyes and lowers his gaze axis by 30° relative to the horizontal plane.

In this case the location of the second adapted point (NV) may be used to determine the near vision part of the optical element and possibly the far vision part of the optical element that are optimal to the subject.

However, the first/second zone, the first/second adapted point and the first/second reference posture may be related to other visual postures than the far/near vision posture, for example it could be an intermediate vision posture. For example, the intermediate vision posture may be gazing a target on a laptop, looking a specific element of a dashboard in a driving situation, manipulating specific object on a table for a specific activity.

In this disclosure one added between bracket the two letters NV (meaning near vision) to elements related to the second zone, the second adapted point and the second reference posture to help the reader understand this disclosure. The only purpose of this indication is to help the reader and not to restrict the scope of the protection as defined in the claims.

The system 101 may comprise a calculation module 102.

The calculation module 102 may comprise a memory 102-a and a processor 102-b.

The calculation module 102 may be a low resource calculation module.

Examples of processors 102-b include microprocessors, microcontrollers, graphics processing units (GPUs), central processing units (CPUs), application processors, digital signal processors (DSPs), reduced instruction set computing (RISC) processors, systems on a chip (SoC), baseband processors, field-programmable gate arrays (FPGAs), programmable logic devices (PLDs), state machines, gated logic, discrete hardware circuits, and other suitable hardware configured to perform the various functionality described throughout this disclosure.

The memory 102-a is computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor 102-a of the calculation module 102.

The calculation module 102 may be included in independent module, for example, a smartphone or a computer, a system on chip (SoC) or a graphics processing unit (GPU). The calculation module 102 may also be a virtual machine located on a cloud network or a server not co-located with the subject or the system 101.

**As** presented in figure 1, and optionally, the system may comprise a capture device 103. The capture device 103 may be an image capture device such as a camera or a camera that may comprise an optical zoom. The capture device may be also an eye tracking device or a head tracking device.

The figure 2 represents an embodiment of the system 101. In this embodiment the system is sensibly a parallelepiped more precisely a rectangular cuboid. The image capture device 103 may be located in the upper region of the system 101. In embodiments, the height off the image capture device 103 may be adapted to the height of the subject.

In embodiments, the system 101 may also be configured to determine or measure values of the parameters of the first reference posture (FV), during an occurrence, by the subject, of the first reference posture (FV).

In embodiments, the system 101 may also be configured to determine or measure values of the parameters of the second reference posture (NV), during an occurrence, by the subject, of the second reference posture (NV).

To realize an occurrence of the first reference posture (FV), the subject may be instructed to gaze naturally at a first target.

To realize an occurrence of the second reference posture (NV), the subject may be instructed to gaze naturally at a second target (NV).

**As** previously, the expressions NV and FV, added between bracket, have for only purpose helping the reader and not restricting the scope of the protection as defined in the claims.

The system 101 may also comprise the first target (FV) and/or the second target (NV) that the subject gazes at during the realization of the occurrence of the first reference posture (FV) and the occurrence of the second reference posture (NV).

**As** previously, the expressions NV and FV, added between bracket, have for only purpose helping the reader and not restricting the scope of the protection as defined in the claims.

The first target (FV) may be for example an object reflected by a two-way mirror 201 (or one-way glass, half-silvered mirror, and semi-transparent mirror) placed on a face of the parallelepiped in the direction of the subject. The two-way mirror positioned may be positioned vertically.

The two-way mirror 201 is a reciprocal mirror that appears reflective on one side and transparent at the other. The perception of one-way transmission is achieved when one side of the mirror is brightly lit, and the other side is dark. This allows viewing from the darkened side but not vice versa.

In this case the image capture device 103 may be located behind this two-way mirror 201 on which the subject will gaze at the first target (FV). The image capture device 103 may be placed such that the position of its principal optical axis relative to the mirror is known. Typically, the camera is arranged such that its principal optical axis extends substantially perpendicularly to the mirror. In this case the object reflected by the mirror 201 can be the face of the wearer watching himself in the mirror, especially it can be the middle of his face, the bridge of the frame, his nose or any landmark that can help him to orient his gaze straight ahead and horizontally, so in the mid-sagittal plane and at eye level.

The first target (FV) may also be a light located just in front of the wearer, at eyes level. This can be more efficient especially for wearer with low acuity who can't distinguish themselves in the mirror.

The second target (NV) may be located on an accessory held by the subject.

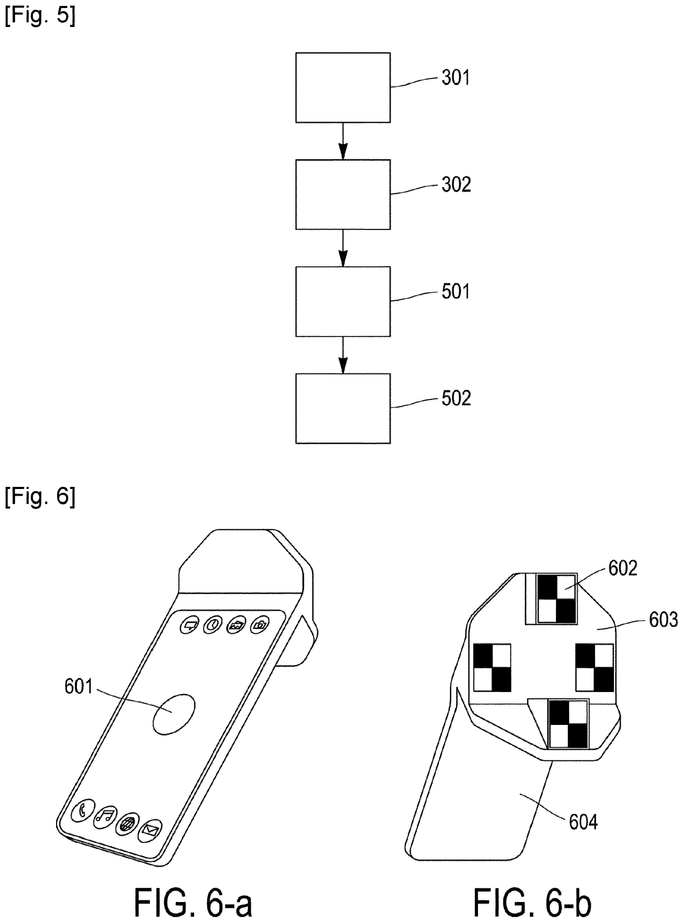

Figures 6-a and 6-b represent an example of the accessory held by the subject. This accessory may have a shape sensibly similar to a shape of a smartphone. Furthermore, this accessory may have a face with a representation of a graphical user interface (GUI) of a smartphone. In the example of the figure 6-a, the circle 601 is a target that the subject is instructed to gaze at while the near vision posture is determined.

In embodiments one can also have an accessory, with the face viewed by the subject, that does not represent a smartphone. In this case the subject may place his own smartphone on the accessory and have a more natural way of holding and gazing at the accessory. The shape of the accessory is then configured to allow the subject's personal smartphone to be placed during the measurement, to obtain even more precise reference postures.

The use of this accessory with the face representing a smartphone or with the real smartphone posed on, allows obtaining a more natural near vision posture representing situations when the subject is using an electronic device.

In embodiments one can also have any tracking device or method to track the screen of a smartphone or a face of a device handled by the subject, meaning tracking the position of a visual target on the face relatively to the head of the subject.

The PCT application reference WO2011161087A1 describes more example of the first and second target that can be used by the skilled person while realizing the object of this disclosure.

The system 101 can also comprise (directly integrated or in a remote device wired or wirelessly linked to the other elements of the system 101) a module for inputting and outputting data/instructions/commands/results from and to a user in charge of running this machine and determining the postures of the subject. This module may comprise a screen, a keyboard and a mouse.

### Description of the method for determining the probability model of the parameter of the first reference posture (FV) of a subject

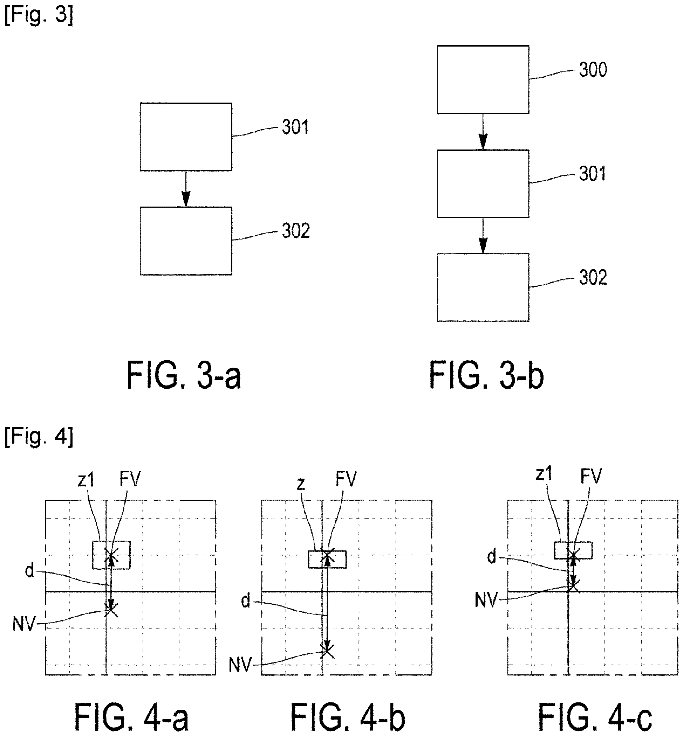

To realize the determination of the probability model of the parameter of the first reference posture (FV) of the subject, the memory 102-a may store a computer program comprising instructions which, when the program is executed by the processor 102-b, cause the control module 102 to carry out a method, for example a computer implemented method, for determining the probability model of the parameter of the first reference posture (FV) of the subject. The probability model of the first reference posture (FV) may be intended to determine the value of the manufacturing parameters of the optical element adapted to the subject. The method, as presented in figure 3-a, comprises:
- a step 301 of determining at least one value of a parameter of a second reference posture (NV), during an occurrence of the second reference posture, and
- a step 302 of determining the probability model of the parameter of the first reference posture based on the at least one value of the parameter of the second reference posture (NV).
- The method, as presented in figure 3-b, comprises:
- a step 300 of determining at least one value of the parameter of the first reference posture (FV) during an occurrence of the first reference posture,
- a step 302 of determining at least one value of a parameter of a second reference posture (NV), during an occurrence of the second reference posture,
- a step 303 of determining the probability model based on the at least one value of the parameter of the second reference posture (NV) and possibly the at least one value of the parameter of the first reference posture (FV).

The parameter of the first reference posture (FV) may be identical to the parameter of the second reference posture (NV).

The parameter of the first reference posture (FV) may be an orientation of a head of the subject relatively to the gaze axis. For example, the orientation may be characterized by a cap angle value of the head and/or a frontal angle value of the head. The parameter of the first reference posture (FV) may also be a parameter of a zone of the optical element across which a gaze axis of the subject passes in the first reference posture (FV). The zone may be a point. For far vision, the frontal angle may be the pantoscopic angle corresponding to the inclination of the head around a frontal axis oriented right/left, that is, an oriented angle which reflects the fact that the subject tends to have his head more or less lifted or lowered when gazing at an object placed in front of him. Panstocopic angle is described in ISO13666.

For near vision posture, the frontal angle may also be an angle made by the frame (or the lenses) of the eyeglasses in relation to the axis of gaze (axis from eye to target or axis from cyclops's eye to -target) around the transverse axis (frontal axis) of the subject. In this case, the inclination with respect to the vertical or horizontal is a particular case of the frontal angle as defined for far vision posture.

The cap angle corresponds to the inclination of the head around a longitudinal axis oriented, that is, an angle which reflects the fact that the subject tends to have his head not perfectly straight but turn more or less to the right or the left when gazing at an object placed in front of him.

The first reference posture (FV) may be a deviated posture of the second reference posture (NV).

Before the beginning of the measure of the first reference posture (FV), one may instruct the subject to stand in a natural position.

One may also determine a first set (FV) of values of the parameter of the first reference posture (FV) using the probability model. The first set can be a continuous or a discrete set of values. For example, when the parameter is a cap angle value of the head or a pantoscopic angle value of the head, the first set may comprise the values for which the likelihood using the probability model is above a threshold.

In other words, the method of the figure 3-a may comprise:
- the determination of a rough probability model associated to the second reference posture (NV) from at least one measure of the second reference posture (NV), advantageously the second reference posture (NV) is different than the first reference posture (FV), furthermore and advantageously the vision correction needed by the wearer is different during the first reference posture (FV) than the second reference posture (NV),
- the determination of the probability model of the first reference posture (FV), will be deduced from the rough probability model of the second reference posture (NV).

The method of the figure 3-b may comprise:
- the determination of a rough probability model associated to the first reference posture (FV) from at least one measure of the first reference posture (FV),
- the determination of a rough probability model associated to the second reference posture (NV) from at least one measure of the second reference posture (NV), advantageously the second reference posture (NV) is different than the first reference posture (FV), furthermore and advantageously the vision correction needed by the wearer is different during the first reference posture (FV) than the second reference posture (NV),
- the determination of the probability model of the first reference posture (FV), that will be an updated version of the rough probability model, deduced from the rough probability model of the second reference posture (NV) and possibly from the rough probability model of the first reference posture (FV).

According to an embodiment, the model of constraints is a parametric model having as parameter at least parameters of the second reference posture. The model of constraints may be a model of design constraints and/or a model of behaviour constraints.

The model of design constraints may be a model based on the design of the optical element intended to be manufactured. In this embodiment, the optical element is a progressive optical element. The parameters of the model of design constraints may be parameters of the progression curve of corridor of the optical element (progression curve means the spatial function of the addition in the progression corridor). For example, the parameters of the progression curve of corridor of the optical element may be a length of corridor of the optical element. The value of the length of corridor may be a standard value (for example 18mm if the parameter is the length of corridor) or an input value (between 10 mm and 20mm or between 14mm or 18mm). The input value may be provided by the ECP, by the lens manufacturer or by the subject.

The model of behaviour constraint may be a theorical model or experimental model based on collected data.

Using the probability model, one may determine values parameters of the optical element that would be allowed for example a zone of the optical element on which one may place the correction associated to the first reference posture (FV). This zone could have a probability of a crossing of the gaze axis during occurrences of the first reference posture (FV) that is superior to a probability threshold.

To determine this zone one can also consider the part of the optical element having the maximum probability of crossing during occurrences of the first reference posture (FV). When there are multiple maxima, one can take an average of those maxima positions to determine the zone.

Otherwise, one can consider a barycentre of a plurality of point on which the probability is above a threshold. This barycentre may be weighted by the probability of each point.

The probability model may be intended to determine the value of the parameter of the first zone (FV) of the optical element. The parameter of the first reference posture (FV) may represent a location of the optical element. For example, the location may correspond to a point having a high probability of a crossing of a gaze axis of the subject during occurrences of the first reference posture (FV).

The parameter may comprise a shape of the first zone (FV), a size of the first zone (FV), a location of a centre of the first zone (FV), an orientation of the first zone (FV).

The centre of a figure also known as the centroid or geometric centre is the arithmetic mean position of all the points in the surface of the figure. The centre may be a maximal probability or a pondered average.

In a more precise embodiment on which the aim is to determine the value of the parameter of the first zone (FV), the method may comprise:
- a step of determining a first real point (FV) of the optical element across which a gaze axis of a subject passes through during an occurrence of the first reference posture (FV),
- a step of determining a second real point (NV) of the optical element across which a gaze axis of a subject passes through during an occurrence of a second reference posture (NV),
- a step of determining a difference between the first real point (FV) and the second real point (NV),
- a step of determining the probability model based on the difference,
- a step of determining the value of the parameter of the first zone (FV) based on the probability model and for example by maximising the probability model, or by selecting values allowing the probability model to be above a threshold.

In this embodiment, the difference may be a distance between the first real point (FV) and the second real point (NV).

In embodiments one may consider that the first zone (FV) has a predefined shape, size and orientation. For example, the first zone (FV) may have a rectangular shape, with a sensibly horizontal orientation and predefined size for example between 2 and 3 mm for the long size of the rectangle and between 0.5mm and 1.5mm for the small size of the rectangle.

By sensibly horizontal we mean that the long size of the rectangle is almost parallel to the longitudinal axis of the optical element, for example to the horizontal centreline of the boxed lens system as described in the ISO13666.

The first zone may have a representing zone. The representing point may be a maximal probability in the zone or a pondered average of the probability in the zone.

The location of the representing point of the first zone (FV) may depend on the difference the first reference posture and the second reference posture. To determine this location, one may compare the difference to a difference threshold.

A representing point of the first zone (FV) may be located in the vicinity of the first real point (FV) when the difference is comprised between a first difference threshold and a second difference threshold.

Thus from at least the determined value of the parameter of the second posture, the probability model takes into account that the first reference posture is not affected by the second reference posture.

The representing point of the first zone (FV) may be located above the first real point (FV) when the difference is lower than the first difference threshold.

Thus from at least the determined value of the parameter of the second posture, the probability model takes into account that the first reference posture is affected by the second reference posture.

The representing point of the first zone (FV) may be located below the first real point (FV) when the difference is higher than the second difference threshold.

Thus from at least the determined value of the parameter of the second posture, the probability model takes into account that the first reference posture is affected by the second reference posture. By below and above one takes the reference of the optical element when this optical element is worn by the subject. For example, if the optical element is a lens, if a first point is above a second point it means that the first point is closer to the upper part of the lens when the lens is worn by the subject.

For example, in the case of frontal angle, the first threshold may be 15° or 10° or less than 10°, the second threshold may be 35° or 40° or more than 40°.

The figures 4-a to 4-b represent examples of the determination of the location of the first zone and of the determination of the localisation of the representing point of the first zone (FV).

**As** represented in figure 4-a, the difference d between the first real point 501 (FV) and the second real point p2 (NV) is comprised between the first difference threshold and the second threshold, therefore the representing point of the first zone z1 (FV) is located in the vicinity of the first real point p1 (FV).

**As** represented in figure 4-b, the difference d between the first real point p1 (FV) and the second real point p2 (NV) is higher than the second difference threshold, therefore the representing point of the first zone z1 (FV) is located below the first real point p1 (FV).

**As** represented in figure 4-c, the difference d between the first real point p1 (FV) and the second real point p2 (NV) is lower than the first difference threshold, therefore the representing point of the first zone z1 (FV) is located above the first real point p1 (FV).

In embodiments the method may also comprise a step of determining, based on the value of the parameter of first zone (FV), the value of the parameters for manufacturing the optical element.

Advantageously, the probability model allows to have new postural information and to make specific recommendations, for example, for lens centration.

In embodiments the system 101 may display information related to the probability model, for example a representation of the first zone (FV) to the Eye Care Professional (ECP). This presentation may be realized using the module for inputting and outputting data/instructions/commands/results.

**The** ECP may select a value of the parameter of the first reference posture (FV) based on the displayed information related to the probability model.

When the first zone (FV) is displayed, the ECP may select one of the points of the first zone (FV) as being a first adapted point (FV).

The value of the parameters for manufacturing the optical element may depend on the location of the first adapted point (FV) or on the value of the parameter of the first reference posture (FV) selected by the ECP.

In other words, in embodiments, the method can also comprise a step of displaying to the ECP the first zone (FV) of the lenses used by the subject in far vision posture. In embodiments the method may also comprise displaying to the ECP a second zone (NV) used by the subject in near vision posture. The system 101 can be configured to allow the ECP to select one of the points of the first zone (FV) as the first adapted point (FV) and/or to select one of the points of the second zone (NV) as a second adapted point (NV).

Using this disclosure, the possibility for the ECP of moving the part of the lenses used by the subject in far vision posture can be limited by the system 101. For example, when the subject gazes down more with their eyes than her/his head in the near vision posture, for example if the subject lower their gaze much more than their head in near vision) the near vision part of the lenses will be further down the lens. Increasing the pantoscopic angle will therefore be limited during the edition and so the possibility to move up near vision part of the lenses. In opposite when the subject gazes down more with her/his head than their eyes in the near vision posture the near vision gaze will be higher up in the lens. Lowering the pantoscopic angle will therefore be limited during the edition and so the possibility to move down near vision part of the lenses.

This embodiment is particularly advantageous when the pantoscopic angle of the subject is not reproducible, meaning is varying during a succession of measurements of the far vision posture (FV). For example, when after gazing at his reflect on the mirror in far vision posture (FV), the subject would not raise his head well or raise it too much. As one wants to limit the number of measures to limit the duration of the process, if the head ports are too different the average is not very meaningful. For example, if one obtains the following pantoscopic angles 8°, 15°, 10° and 13, one can hesitate between several ways of averaging these values:
- overall average: 11.5°
- average excluding 8° as a suspect measurement: 12.7°
- average excluding 15° as a suspect measurement: 10.3°
- low values are favoured: 9°
- high values are favoured: 14°

It is difficult to find an effective criterion for making the right choice, and yet in this case there is a good chance that certain bad postures measured will degrade the optimal posture and then have a discomfort of the subject while he later uses the optical element.

To limit this, one can advantageously take into account the near vision posture (NV) also to determine the far vision part of the lenses, especially the near vision posture (NV) can give information, for example constraints on the far vision posture of reference (FV).

If in near vision posture (NV), the gaze axes projection is getting closer to the far vision gaze axes projection, it is likely that the subject had raised their head too much on a certain far vision measurement. One can favour the high values of the pantoscopic angle (from 11.5° to 15° in our example, one could even suggest 14°).

If in near vision posture (NV), the gaze axes projection is very low on the lenses compared to the far vision projection point, it is likely that the subject has not straightened up enough on a certain far vision measurement. One can favour the low values of the panto values (from 8° to 11.5°, one can suggest 9° rather than 11.5° by default).

In order to have a better interpretation of the near vision posture (NV), one can use multiple parameters related to the near vision posture (NV), such as near vision heights, meaning height of the gaze projection on the lens in boxing coordinates in near vision posture (see ISO 13666), or vertical boxed lens size (see ISO 13666).

For example, in the case where in near vision posture (NV), the gaze axes projection is getting closer to the far vision gaze axes projection, one of the multiple parameters could be near vision heights, meaning height of the gaze projection on the lens in boxing coordinates in near vision posture. If this parameter is high it means that there is space on the lenses if the user want to lower more is gaze for near vision so one could keep average position for far vision posture (FV), or if the position of the projection of his gaze in near vision is too low and constrained by the frame circles so one should favour the higher values of the pantoscopic angle.

The use of vertical boxed lens size is similar to the near vision height, meaning that one can know if there is space on the lens if the user wants to upper more his gaze for far vision so one could favour the higher values of the pantoscopic angle.

In other embodiments, the calculation module 102 may select the first adapted point (FV). The calculation module 102 may be configured to select the representing point of the first zone (FV) as the first adapted point (FV).

In an embodiment presented figure 5 the method may also comprise:
- a step 501 of measuring a plurality of values of a parameter of a third reference posture (for example postures used for far vision) by repeating at least twice:
- a step 502 of measuring at least one value of the parameter of the third reference posture (FV) during a plurality of occurrences of the third reference posture (FV).

In this embodiment, the probability model of the first reference posture (FV) may also be determined based on the plurality of the values of the parameter determined during the plurality of occurrences of the third reference posture (FV).

One may average the plurality of the values to obtain the probability model of the first reference posture (FV).

In an embodiment, the method may comprise applying weights to the plurality of the values, the weights depending on indications of a relevance associated respectively with the occurrence of the third reference posture and types associated respectively with the deviated postures of the first reference posture.

In embodiments the method of the figure 3 or the figure 5 may comprise the determination of a confidence level of the first reference posture (FV) and/or a confidence level of the second reference posture (NV).

**In** an embodiment the method of figure 3 and figure 5 may comprise a step of determining the pantoscopic angle value and the cap angle value characterizing the first reference posture (FV) by means of conditional probabilities depending on a previous occurrence of the first reference posture (FV) and/or a previous deviated posture of the previous occurrences of the first reference posture (FV), and/or a step of determining the pantoscopic angle value and the cap angle value characterizing the second reference posture (NV) by means of conditional probabilities depending on a previous occurrence of the second reference posture (NV) and/or a previous deviated posture of the previous occurrences of the second reference posture (NV).

The method may also comprise the use of a model for example a model of human behaviour that can be advantageously coupled to an a priori model.

This model can take the form of conditional probabilities, for example the probability of having an optimal reference posture pₒ, knowing that there is a unique posture pᵤ or a sequence of postures pᵢ measured during i iterations, and knowing the types of deviation used (gazing up, left, reading a document, etc.) prior to the unique posture pᵤ or each posture pᵢ. For example, studies have shown that the probability that the cap of the head is good is stronger once the head has been turned right then left than prior to these movements. The measurements of the cap obtained after these movements will therefore have a higher weight in calculations of this value. Similar considerations can be made advantageously for the pantoscopic angle after the ceiling has been gazed at.

The model of human behaviour could use one of the mathematical tools listed below:
- conditional probabilities, for example the probability of having a reference posture p knowing that there is a unique measured posture pᵤ or a sequence of measured postures pᵢ measured during i iterations and depending on the types of deviation used (gazing up, left, reading a document, etc.) prior to each measured posture pᵢ: these conditional properties could be organised in a Markov chain.
- advanced analyses for detecting whether a value of a posture is coherent or aberrant and accordingly censuring aberrant values.

The PCT application reference WO2011161087A1 describes more examples of the human behaviour model and the a-priori model that can be used in the object of this disclosure and the skilled person will know how to implement these models in the object of this disclosure.

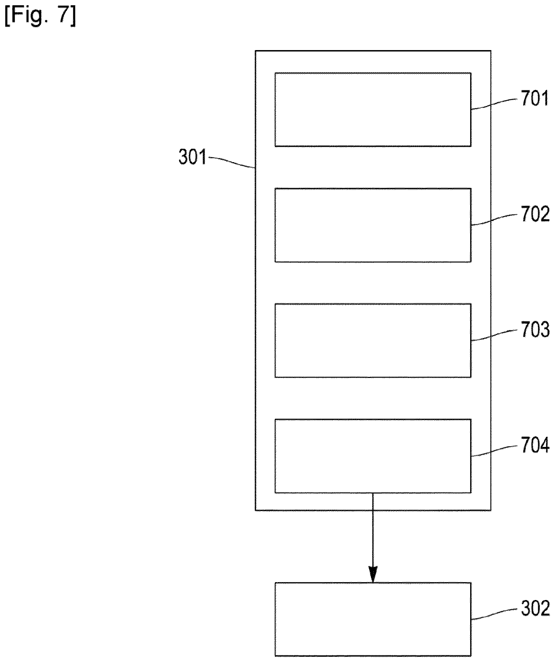

As presented in figure 7 the step 301 may also comprise:
- a step 701 of obtaining a first image of the subject during an occurrence of the first reference posture (FV) and
- a step 702 of measuring a location of singular points on the first image, and/or
- a step 703 of obtaining a second image of the subject during an occurrence of the second reference posture (NV) and
- a step 704 of measuring a location of singular points on the second image.

The first image and the second image may be obtained from the image capture device 103.

The singular points of the first image and/or the singular points of the second image may be supported by an accessory fixed on an eyeglasses frame worn by the subject and/or an accessory held by the subject. The accessory held by the subject may be the same as the accessory on which the first target may be located. The accessory fixed on an eyeglasses frame may be a clip. For more details on the system, reference could be made for example to applications references FR-2860887A and FR-2892529A.

When the singular points are supported by the accessory represented in the figures 6-a and 6-b these singular points can be markers. These markers may be located in an extremity of the accessory opposite to the subject. As presented in the figure 6-b the markers 602 can be located sensibly on a plane 603 having an angle comprise between 90° and 30° with a plane 604 of the accessory representing the smartphone. The markers may be mini checkerboards. This plan 603, in addition to making it possible to fix the markers on the tablet, makes it possible to visually isolate the markers from the environment (the checked shirt for example). This way, on the camera image, there are no mini checkerboards between the tablet/phone markers. However, depending on its size and orientation, this plane can shade or even hide the markers, thus impacting their visibility on the camera image. Its inclination, shape and size can therefore influence the quality of the detection of the markers.

The markers aim to determine the localisation and the orientation of the accessory. The skilled person would know how to make this determination using the markers; he may use machine learning, a neural network, or a Lidar.

The accessory contains at least three singular points, as three singular points is the minimum to obtain the complete 3D position of an object. Advantageously one can add a fourth marker to obtain a more robust detection (less confusion with parasitic elements on the image, for example a checked shirt carried by the subject) and more precise localisation of the object. For example, when three markers are approximately at the same distance from the camera, the orientation of the object may be not precise enough. In this case the fourth marker may help improve this precision. To also improve the precision of the localisation, one can use four non-coplanar markers. The skilled person would know how to determine the localisation and orientation of the object based on a picture on which at least three markers are represented.

The inclination of the markers may be configured to obtain the best oriented possible localisation estimation in relation to the camera. To achieve it is advantageous that the markers are clearly visible on the image, and this for the whole expected positions of the accessory. Depending on the subject's preferences, the accessory can be held close to vertical or close to horizontal, or even be oriented slightly to the right or left.

Advantageously one can group the markers in a compact manner, to allow the markers to fit across the width of a smartphone and not stretch it out too much or unbalance the accessory. This allows having a less bulky accessory and that can be apprehended as a smartphone by the subject. The size is of great importance because it influences the behaviour of the subject, in particular the possibility of holding the accessory with one hand and placing it as if it were a real smartphone. Reducing the spacing between the right-left markers therefore improves the grip of the accessory by the subject.

To improve the precision of the reference postures and more precisely improve the precision of the gaze axis of the subject with relation to the frame, the position of the target on which the subject gazes at must be precise and accurate.

For more details on the link between the markers and the parameter of the reference posture system, reference could be made for example to application reference FR-2974722A.

The method presented figure 7 may comprise the control of a zoom factor of the optical zoom of the image capture device 103.

To control the zoom factor the method of the figure 7 may comprise:
- a step of controlling the zoom factor so when the first image is taken by the camera, a field of view of the camera comprises the head and an accessory held by the subject,
- a step of controlling the zoom factor so when the second image is taken by the camera, the field of view of the camera only comprises the head of the subject.

By way of variant, it is not necessary to start out from an image. One may use a system for tracking the position of the head in real time (or delayed calculation).

By way of variant, one may use a system for tracking the position of the eye in real time (or delayed calculation) relatively to the frame.

Examples of systems for tracking the position of the head can be the following, in a non-limiting manner:
- An accelerometer placed fixed relative to the head. After appropriate calibration continuous acquisition of the acceleration produces tracking of the position of the head.
- An ultrasound system.
- Infrared transmitters placed on the head of the subject, for example on the accessory fixed on the eyeglasses frame worn by the subject of the type described previously or on any object fixed relative to the head, linked to an infrared camera.
- Any system of three-dimensional reconstruction of the face for obtaining a posture parameter sufficiently precise for obtaining at least the cap angle and the pantoscopic angle. This can be three-dimensional reconstruction of salient points of the face by stereoscopy (or photogrammetry) by means of two cameras (or reconstruction with a single camera linked to scaling means sufficiently precise for study (the scaling means can be for example a known distance between two of the salient points identified on an image).

### Description of a use case of this disclosure

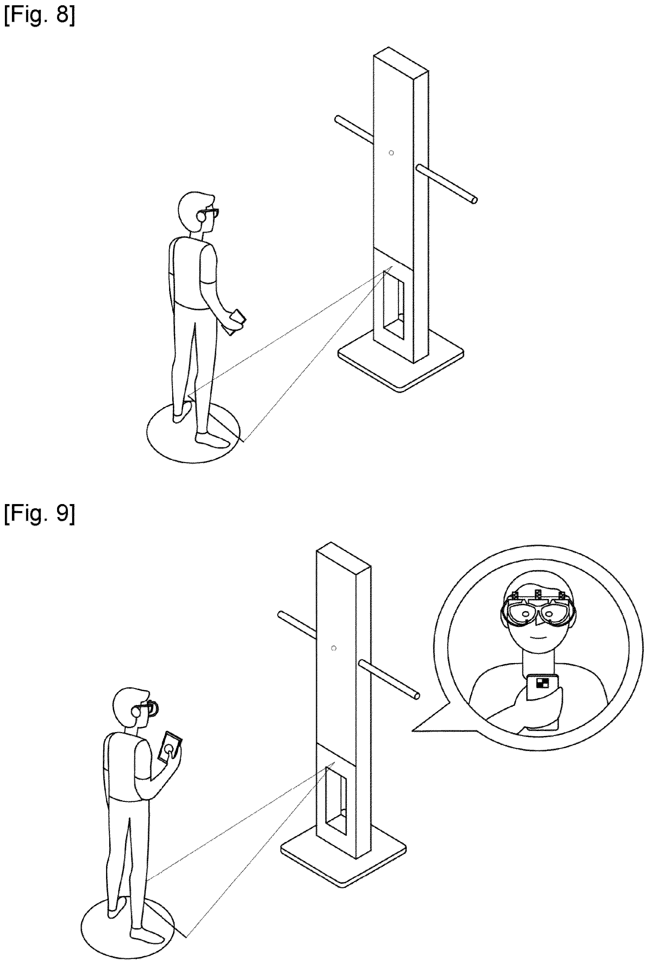

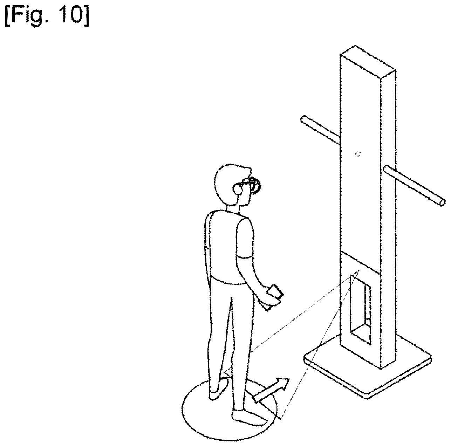

One way of using the object of this disclosure can realized by following the phases below:
- In a first phase the subject stands in front of the mirror of the system 101, at about 150 cm. The figure 8 represents this first step.
- In a second phase, the zoom factor of the image capture device 103 behind the two-way mirror 201 is adjusted to have a precise and reliable measurement of the head posture of the subject.
- In a third phase, a height of a light behind the mirror (forming a visual target) may be adjusted to the exact height of the wearer's eyes. Because the position of the light against the camera is known (fixed on it in our implementation) it's possible to calculate and display a line on the image obtained from the image capture device 103 representing the light height.
- In a fourth phase, the subject is asked to watch a light. One can also use other types of targets to direct the gaze of the subject in the direction required for the reference posture. For example, this can be a light placed at eye level or the subject can look at himself in the mirror (he looks at the centre of his face, between his two eyes or the bridge of the frame if he can distinguish them). Then one may measure a first far vision posture.
- In a fifth phase, the subject is asked to watch a target on the accessory that he holds, for example a phone. Then one may measure the head posture of the head (actually the clip positioned on the wearer's frame).
- In a sixth phase, the zoom factor of the of the image capture device 103 is adjusted to visualize the head at the same time as the wearer's smartphone. This is represented in the figure 9.
- In a seventh phase, one can then measure using an image provided by the image capture device 103 the position of the smartphone and the wearer's frame against the camera.
- In an eighth phase, one can estimate a near vision posture using the measures realized in the phases five and seven.
- In a nineth phase and optionally, a second measurement of the far vision posture can be made following the phases 2, 3 and 4.
- In a tenth phase the subject is asked to come forward at 80 cm from the two-way mirror 201. This phase is represented in figure 10.
- In an eleventh phase, one can determine an updated version of the far vision posture using for example the method describe in the PCT application reference WO2011161087A1. We have the following specification for this protocol:
   ∘ Phases 1 to 4 can be considered as the first posture of the measurement.
   ∘ The phase 10 is considered as a diversion (like "watch downside")
   ∘ One can reduce the number far vision posture (already done during phases 4 and 9) and diversions (during phases 5 and 10)
   ∘ Then stereoscopic measurements can be done for far vision (ERC, PD, FH, frame warp...).

## Claims

1. A calculation module (102) for determining a probability model of a parameter of a first reference posture (FV) of a subject,
the probability model being intended to determine at least a production parameter of an optical element suitable for the subject,
the calculation module (102) being configured:
- to determine (301) at least one value of a parameter of a second reference posture (NV), during an occurrence of the second reference posture (NV), and
- to determine (302) the probability model of a parameter of a first reference posture (FV) based on the at least one value of the parameter of the second reference posture (NV) and based on a model of constraints.

2. The calculation module (102) according to the claim 1, wherein the calculation module (102) is further configured to determine (300) at least one value of the parameter of the first reference posture (FV) during an occurrence of the first reference posture (FV), and
wherein determining (302) the probability model is based on at least one value of the parameter of the first reference posture (FV) and the at least one value of the parameter of the second reference posture (NV).

3. The calculation module (102) according to any precedent claims, the model of constraints is a model of design constraints and/or a model of behaviour constraints.

4. The calculation module (102) according to any precedent claims, the first and/or second reference posture (FV) being **characterized by** an orientation of a head of the subject relatively to a gaze axis of the subject.

5. The calculation module (102) according to the claim 4,
the parameter of the first and/or the second reference posture being a cap angle value and/or a frontal angle value.

6. The calculation module (102) according to anyone of the claims 1 to 5, the parameter of the first and/or second reference posture being a location of the optical element crossed by a gaze axis of the subject while respectively the first and/or second reference posture.

7. The calculation module (102) according to claim 2 combined with claims 5 or 6 wherein the step of determining the probability model is based at least on the difference between the first reference posture and the second reference posture.

8. The calculation module (102) according to anyone of the claims 1 to 7, wherein the calculation module (102) is further configured, based on the probability model, to determine a zone of at least one parameter of the first reference posture, the values of the at least one parameter of the first reference posture being superior to a given probability threshold.

9. The calculation module (102) according to anyone of the claims 1 to 8,
wherein the calculation module (102) is further configured, based on the probability model, to determine a representing point, the representing point being a point with a maximal probability or a pondered average of a plurality of points.

10. The calculation module (102) according to claim 2 combined with the claims 7, 8 and 9 or claims 7 and 9,
- the representing point (FV) being located in the vicinity of the first reference posture (FV) when the difference is comprised between a first difference threshold and a second difference threshold,
- the representing point (FV) being located above the first reference posture (FV) when the difference is lower than the first difference threshold,
- the representing point (FV) being located below the first reference posture (FV) when the difference is higher than the second difference threshold.

11. The calculation module (102) according to the claim 10 and 8,
the calculation module being configured to obtain the location of an adapted point (FV) by selecting an adapted point (FV) among points of the zone (FV).

12. The calculation module (102) according to any one of the claims 1 to 11, being also configured
to obtain a first image of the subject during the occurrence of the first reference posture (FV) and
to measure a location of singular points on the first image, and/or
to obtain a second image of the subject during the occurrence of second first reference posture (NV) and
to measure a location of singular points on the second image, and
wherein the step of determining the value of the parameter of the first and/or second reference posture is based on the measured location of the singular points.

13. The calculation module (102) according to the claim 12, in which the singular points of the first image and/or the singular points of the second image being supported by an accessory fixed on an eyeglasses frame worn by the subject and/or an accessory held by the subject.

14. System for determining a probability model of a parameter of a first reference posture (FV) of the subject, the probability model being intended to determine at least a production parameter of an optical element suitable for the subject,
the system comprising:
• a capture device to measure a second reference posture,
• a calculation module configured
- to determine at least one value of a parameter of the measured second reference posture (NV), and
- to determine the probability model of a parameter of a first reference posture (FV) based on the at least one value of the parameter of the second reference posture (NV).

15. A method for determining a probability model of a parameter of a first reference posture (FV) of the subject, the probability model being intended to determine at least a production parameter of an optical element suitable for the subject,
the method comprising:
- measuring at least one value of a parameter of a second reference posture (NV),
- determining the probability model of a parameter of a first reference posture based on the at least one value of the parameter of the second reference posture (NV).
